# EUROPÄISCHE PATENTANMELDUNG

(11) **EP 2 322 087 A2**
(43) Veröffentlichungstag der Anmeldung: **18.05.2011**
(21) Anmeldenummer: 10008813.7
(22) Anmeldetag: 24.08.2010
(51) Int. Cl.: A61B 5/022

(54) **Vorrichtung zum Messen von kardiologischen Parametern eines Probanden**

(30) Priorität: 17.11.2009 DE 102009053616
(71) Anmelder: Bosch + Sohn GMBH & CO. KG, 72417 Jungingen (DE)
(72) Erfinder: Welte, Wolfgang, 72351 Geislingen (DE)
(74) Vertreter: Müller - Hoffmann & Partner

(57) **Zusammenfassung**

Eine erfindungsgemäße Vorrichtung erfasst eine Serie von Blutdruckwerten aufeinander folgender Pulsschläge eines Probanden und erstellt daraus ein Oszillationsprofil, das systolische und diastolische Blutdruckwerte sowie weitere kardiologisch relevante Parameter bereitstellen kann, da auch Extrasystolen oder etwa schwankende Amplituden der Druckwerte in dem Oszillationsprofil erfasst werden.

## Beschreibung

Die Erfindung betrifft eine Vorrichtung zum Messen von kardiologischen Parametern eines Probanden. Ferner betrifft die Erfindung eine derartige Vorrichtung, bei der ein Messergebnis auf ein in eine Druckmanschette der Vorrichtung eingegebenes Volumen normierbar ist.

Blutdruckmessungen sind bereits auf vielfältige Weisen durchführbar. Direkte Messungen werden häufig mit Drucksensoren durchgeführt, die nach einer Gefäßpunktion unmittelbar in einer Arterie angeordnet werden. Dies erfolgt somit stets invasiv. Indirekte Blutdruckmessungen sind dagegen meist nicht-invasiv und werden am häufigsten auskultatorisch, palpatorisch oder oszillatorisch bzw. oszillometrisch durchgeführt, was jeweils manuell oder automatisiert erfolgen kann.

Bei einer automatisierten oszillatorischen Messung wird der Blutdruck eines Probanden meist mithilfe eines Drucksensors an einer seiner Extremitäten sensorisiert. Pulsatile Druckänderungen in den Arterien nahe der Manschette erzeugen periodische bzw. oszillierende Druckänderungen an der Manschette. Diese Oszillationen an der Manschette werden über weiches Gewebe an den Drucksensor weitergegeben und dort als indirektes Maß für pulsatile Druckänderungen in den unter der Manschette liegenden Arterien zum Messen des Blutdrucks herangezogen. Hierbei wird in der Regel die Pulsfrequenz ermittelt und die systolischen und diastolischen Blutdrücke werden ermittelt.

Der Erfindung liegt die Aufgabe zugrunde, eine Vorrichtung zum Messen des Blutdrucks eines Probanden anzugeben, die neben dem Blutdruck auch weitere für eine umfassende Diagnose geeignete kardiologisch relevante Parameter des Probanden erfassen kann, ohne dass dafür weitere Sensoren bereitgestellt werden müssen. Ferner liegt der Erfindung die Aufgabe zugrunde, eine Vorrichtung zum Messen von kardiologischen Parametern anzugeben, mit der zuverlässige Aussagen über kardiologische Parameter mit einer hohen Reproduzierbarkeit möglich sind.

Eine erfindungsgemäße Vorrichtung zum Messen von kardiologischen Parametern eines Probanden weist wenigstens eine aufblasbare Manschette auf, die an einer Extremität, beispielsweise an einem Oberarm oder einem Knöchel eines Probanden anlegbar ist. Die Manschette kann dabei an der entsprechenden Extremität festgeschnallt, angeklettet oder in sonstiger Weise angebracht werden. Gemäß einer weiteren Ausführungsform kann die Vorrichtung vier Manschetten aufweisen, wovon jeweils zwei an den Oberarmen und zwei an den Knöcheln eines menschlichen Probanden anlegbar sind.

Eine Manschette kann wenigstens einen mit einem Gas oder einer Flüssigkeit füllbaren Hohlkörper aufweisen, wodurch die Manschette aufblasbar bzw. aufpumpbar ist, um dadurch einen mechanischen Druck auf die jeweilige Extremität auszuüben. Für unterschiedliche Größen bzw. körperliche Konstitutionen von Probanden können entsprechende Manschetten in verschiedenen Größen und Umfängen gefertigt sein, um deren Anwendung beispielsweise für erwachsene Probanden verschiedener Statur ebenso wie für Kinder zu ermöglichen. Selbstverständlich kann die Vorrichtung und somit auch eine derartige Manschette auch für nicht-menschliche Probanden ausgelegt sein, wie etwa für Haus- oder Nutztiere.

Die Vorrichtung weist zudem wenigstens einen Drucksensor sowie eine Zentraleinheit auf, die mithilfe des Drucksensors eine oszillometrische Messung von Blutdrücken des Probanden vornehmen kann. Dabei kann eine Manschette mit einem daran angeordneten Drucksensor vorgesehen sein, so dass der Drucksensor eine Druckmessung an der entsprechenden Extremität vornehmen kann. Die Manschette kann aber auch einen Druckübertrager aufweisen, mit dem ein in der Zentraleinheit angeordneter Drucksensor verbunden ist, so dass die Druckmessung nicht direkt an einem Sensor an der Manschette erfolgt, sondern davon ausgelagert. Hierzu kann etwa ein pneumatischer Schlauch zur Druckübertragung von der Extremität bzw. der Manschette an den Sensor verwendet werden, wobei ein Puls eine mechanische Schwingung an die Manschette weitergibt, die wiederum über den Schlauch an den Sensor in der Zentraleinheit weitergegeben wird, wo der Puls in Form von Druck bzw. Druckänderungen erfasst wird. Wenn mehrere Manschetten bereitgestellt werden, so ist jeweils wenigstens ein Sensor je Manschette vorgesehen, so dass beispielsweise jede Manschette einen Drucksensor aufweist, mithilfe dessen jeweils ein Blutdruck an der entsprechenden Extremität oszillometrisch messbar ist. Es ist auch eine Ausführungsform der Vorrichtung möglich, die für mehrere Manschetten mit Sensoren ausgelegt ist, aber auch nur mit einer Manschette benutzbar ist, so dass die weiteren Manschetten optional einsetzbar sind. Im Folgenden wird der Einfachheit halber eine Ausführungsform mit einer Manschette und einem Drucksensor betrachtet, wobei jedoch stets auch Ausführungsformen mit mehreren Manschetten bzw. entsprechend mehreren Drucksensoren möglich sind.

Der Drucksensor der Vorrichtung kann beispielsweise ein Passivdrucksensor, ein Relativdrucksensor, ein Absolutdrucksensor oder ein Differenzdrucksensor sein. Dabei kann der Drucksensor ein elektronischer Drucksensor sein. Möglich ist der Einsatz von piezoresistiven, piezoelektrischen, frequenzanalogen, kapazitiven, induktiven, Hallelement-basierten oder weiteren elektronischen Drucksensoren. Wichtig ist hierbei, dass eine oszillometrische Messung durchführbar ist.

Pulsatile Druckänderungen in den Arterien unter der Manschette erzeugen periodische bzw. oszillierende Druckänderungen, die sich über Muskel- und Hautgewebe an die Manschette bzw. den Drucksensor propagieren. Die Oszillationen am Sensor werden dort als indirektes Maß für pulsatile Druckänderungen in den unter der Manschette liegenden Arterien zum Messen des Blutdrucks herangezogen. Hierbei kann eine Differenz zwischen dem systolischen Druck und dem diastolischen Druck aus den gemessenen maximalen Amplitudenwerten in der Zentraleinheit errechnet werden sowie der systolische und der diastolische Blutdruck mithilfe des Drucksensors einzeln ermittelt werden. Darüber hinaus sind auch noch weitere kardiologische Parameter mit der Vorrichtung messbar.

Durch ein Aufpumpen der Manschette der Vorrichtung kann eine Arterie okkludiert, also so weit zugedrückt werden, dass sie nahezu verschlossen ist. Da der Puls schon proximal der okkludierten Arterie auf die aufgepumpte Manschette stößt, treten Oszillationen bei einer Entlastung der Manschette durch einen Druckabfall darin noch oberhalb des systolischen Drucks auf. Während einer Blutdruckmessung mit der Vorrichtung zeigt ein kräftiger Anstieg der Oszillationsamplituden den Zeitpunkt an, zu welchem die Arterie wieder geöffnet ist und die arterielle Durchblutung wieder einsetzt. Der zu diesem Zeitpunkt gemessene Blutdruck entspricht dem systolischen Blutdruck. Danach werden die gemessenen Oszillationsamplituden zunehmend größer und erreichen dann ein Maximum, welches dem arteriellen Mitteldruck entspricht. Dieser arterielle Mitteldruck stellt ein Maß für den insgesamt aufrechterhaltenen Blutstrom in der Peripherie dar. Anschließend werden die gemessenen Oszillationsamplituden wieder kleiner und verschwinden schließlich, wenn die Manschette nahezu entleert ist und somit keinen Druck mehr auf Blutgefäße ausübt.

Anstatt lediglich den systolischen und den diastolischen Druck zu messen, um diese Daten anzeigen zu lassen, erstellt bei der erfindungsgemäßen Vorrichtung die Zentraleinheit eine ganze Serie von Messwerten während des Druckabfalls in der Manschette und kann diese Daten in einer Speichereinheit der Vorrichtung abspeichern. Somit liegt für einen Messvorgang an einer Extremität eine Amplitudenserie vor, welche etwa mittels einer Anzeigeeinheit für einen Benutzer sichtbar angezeigt werden kann. Diese als Oszillationsprofil bezeichnete Amplitudenserie enthält beispielsweise diskrete Messwerte der gemessenen Drücke für jeden Pulsschlag oder kontinuierlich gemessene Druckverläufe während einer zuvor festgelegten Zeitdauer.

Ein Oszillationsprofil enthält somit die Werte des systolischen und des diastolischen Drucks sowie Druckwerte weiterer Pulsschläge eines Messvorgangs und kann somit weitere kardiologisch relevante Informationen vermitteln. Beispielsweise können Extrasystolen, etwa durch Doppelschläge oder Schlag-Aussetzer des Herzens oder ein unregelmäßiger Pulsschlag in Druck oder Frequenz erfasst und ausgegeben werden, so dass eine Diagnose über den kardiologischen Zustand des Probanden anhand von wesentlich mehr relevanten Parametern möglich ist, als bei einer herkömmlichen, lediglich für die Extremwerte sowie die Pulsfrequenz ausgelegten Blutdruckmessung.

Ein Oszillationsprofil soll die Druckamplituden aufeinander folgender Pulsschläge erfassen, muss dabei aber nicht zwangsläufig während eines Druckabfalls in der Manschette erfasst werden. Es ist ebenso möglich, dass der Druck in der Manschette während eines Messvorgangs ansteigt oder ansteigt und wieder absinkt. Mit den Daten der entsprechenden Oszillationsprofile können sich weitere Aussagen über kardiologische Zustände oder Pathologien des peripheren Gefäßsystems treffen lassen.

Hierzu kann die Zentraleinheit mit einer Pumpeinheit verbunden sein, welche derart ansteuerbar ist, dass die Pumpeinheit die Manschette entsprechend einem gewünschten Druckverlauf aufpumpt oder den Druck darin verringert. Während diesen Druckänderungen werden dann Blutdruckwerte ermittelt und zu einem Oszillationsprofil zusammengefasst. Beispielsweise kann die Zentraleinheit die Pumpeinheit so ansteuern, dass letztere den Druck in einer zunächst leeren Manschette gleichmäßig erhöht, bis in der Manschette ein gewünschtes Volumen vorliegt oder bis ein gewünschter Druck auf die entsprechende Extremität des Probanden ausgeübt wird. Anschließend wird der Druck in der Manschette langsam verringert. Während dessen nimmt die Zentraleinheit die gemessenen Blutdruckwerte von aufeinander folgenden Pulsschlägen des Probanden auf und erstellt ein entsprechendes Oszillationsprofil.

Gemäß einer Ausführungsform weist die Vorrichtung eine Speichereinheit auf, die mit der Zentraleinheit verbunden ist, so dass ein oder mehrere erstelle Oszillationsprofile in der Speichereinheit speicherbar sind.

Vorzugsweise werden für ein Oszillationsprofil eines Messvorgangs die Druckamplituden aufeinander folgende Pulsschläge erfasst. Es ist aber auch möglich, die Vorrichtung so zu betreiben, dass lediglich jeder zweite Pulsschlag erfasst wird oder die Pulsschläge nach anderen gewünschten Mustern erfasst werden. Dies kann beispielsweise dann eingesetzt werden, wenn resultierende Datensätze einen geringen Speicherbedarf haben sollen.

Gemäß einer weiteren Ausführungsform erfasst die Vorrichtung für ein Oszillationsprofil den systolischen sowie den diastolischen Blutdruck und daneben lediglich die solchen Pulsschlägen zugeordneten Druckamplituden, die unregelmäßig aufeinander folgen. Beispielsweise kann dafür ein Toleranzbereich für eine Abweichung von einer durchschnittlichen Pulsfrequenz bestimmt werden, so dass bei einer Abweichung davon, etwa durch einen Pulsschlag einer Extrasystole, ein Doppelschlag erfasst wird, wohingegen weitere Pulsschläge, die wieder mit einer im Toleranzbereich liegenden Frequenz aufeinander folgen, nicht erfasst werden.

Gemäß einer weiteren Ausführungsform weist die Vorrichtung eine Anzeigeeinheit auf, welche ein Oszillationsprofil anzeigen kann. Es sind akustische Anzeigen oder Anzeigen per Vibrationen möglich, vorzugsweise erfolgt das Anzeigen allerdings grafisch. Dazu kann die Anzeigeeinheit etwa ein LCD-Display, ein Plasmadisplay oder ein organisches-LED-Display sein und ein Oszillationsprofil in Form von Kurvenverläufen, Balkendiagrammen oder Zahlentabellen einem Benutzer anzeigen.

Anstatt der Anzeigeeinheit oder alternativ dazu weist eine Vorrichtung gemäß einer weiteren Ausführungsform eine Ausgabeeinheit auf, mit der die Oszillationsprofile ausgegeben werden können. Beispielsweise kann ein externer Monitor an die Ausgabeeinheit angeschlossen werden, so dass die Vorrichtung Grafikdaten zum Darstellen der Oszillationsprofile über die Anzeigeeinheit an den Monitor ausgeben kann.

Eine hier beschriebene automatisierte oszillatorische Messung mithilfe der Vorrichtung bedarf üblicherweise nicht der Durchführung durch einen Arzt oder einen sonstigen Spezialisten und kann daher auch von nicht speziell qualifizierten Personen durchgeführt werden. Somit kann eine erfindungsgemäße Vorrichtung auch von einer Arzthelferin oder einem Arzthelfer eingesetzt werden. Der Einsatz der Vorrichtung kann aber auch vollautomatisch durch eine Maschine bzw. einen Rechner, wie beispielsweise eine automatisierte Patientenüberwachung in einer Klinik, oder an einem Wohnsitz eines Probanden geschehen.

Wie bereits beschrieben, kann die Vorrichtung gemäß einer weiteren Ausführungsform vier Manschetten und vier Drucksensoren aufweisen. Mit jeweils drei Manschetten und drei Sensoren kann dabei auch ein ABI-Wert (aus engl. Ankle-Brachial-Index, Knöchel-Arm-Index) ermittelt werden. Somit kann diese Vorrichtung etwa zwei ABI-Werte, jeweils einen systolischen und einen diastolischen Blutdruck je Manschette sowie vier entsprechende Oszillationsprofile messen bzw. erstellen und für eine darauf aufbauende Diganose bereitstellen.

Gemäß einer weiteren Ausführungsform der Erfindung ist die Vorrichtung dazu geeignet, eine Profilhöhe eines Oszillationsprofils auf ein Volumen in der Manschette zu normieren. Dies bedeutet, dass die Höhe der Amplituden eines Oszillationsprofils in Abhängigkeit eines Manschettenvolumens streckbar, verlängerbar, stauchbar oder verkürzbar sind.

Dazu weist die Vorrichtung nach einer weiteren Ausführungsform eine Anpasseinheit zum Bestimmen eines Volumens in der Manschette und zum Normieren einer Amplitudenhöhe des Oszillationsprofils entsprechend des Volumens auf. Die Anpasseinheit kann einen von dem Volumen abhängigen Korrekturfaktor errechnen und die Amplitudenhöhe des Oszillationsprofils damit multiplizieren, um ein korrigiertes Oszillationsprofil zu erstellen. Die Anpasseinheit kann auch in der Zentraleinheit enthalten sein oder mit dieser identisch sein. Um das Volumen in der Manschette zu messen, welches in diese hinein gegeben wurde, weist die Vorrichtung vorzugsweise einen Volumensensor auf. Das Volumen kann aber auch indirekt bestimmt werden, etwa mithilfe eines Manschettendrucksensors zum Sensorisieren eines Innendrucks in der Manschette oder eines Drucks, der von der Manschette auf die entsprechende Extremität ausgeübt wird, und eines Zeitmessers zum Messen einer Dauer eines Aufpumpvorgangs der Manschette. Bei einer bekannten Aufpumpgeschwindigkeit aufgrund eines konstanten Volumenflusses des Gases oder der Flüssigkeit in die Manschette hinein kann anhand dieser Messdaten des Zeitmessers und/oder des Innendrucksensors das in der Manschette vorliegende Volumen bestimmt werden.

Für ein bestimmtes Normvolumen liegen bei dieser Ausführungsform in der Zentraleinheit genaue Charakteristiken über Amplitudenhöhen der Oszillationsprofile als abgespeicherte Datensätze vor, die einer durchschnittlichen Stärke bzw. Dicke einer Extremität entsprechen können. Das ermittelte tatsächliche Volumen in der Manschette kann insbesondere dann davon abweichen, wenn die Extremität, an der eine Messung erfolgen soll, dicker oder dünner als durchschnittlich ist oder wenn die Manschette stärker oder weniger stark aufgepumpt wurde, als bei einer vorangehenden Messung an derselben Extremität.

Eine relativ dünne Extremität bzw. eine locker sitzende Manschette bedarf eines ein hohen Manschettenvolumens, um eine Arterie zu okkludieren. Die Amplitudenhöhe in einem dazugehörigen Oszillationsprofil kann dann verhältnismäßig klein sein. Andererseits benötigt eine relativ dicke Extremität bzw. eine straff sitzende Manschette nur ein vergleichsweise geringes Aufpumpvolumen in der Manschette. Die Amplitudenhöhe in einem entsprechenden Oszillationsprofil ist in diesem Fall relativ hoch. Derartige Abweichungen in Oszillationsprofilen können deren Aussagekraft beeinträchtigen und Rückschlüsse über mögliche Pathologien erschweren. Allerdings kann mithilfe der Vorrichtung das Volumen in der Manschette direkt gemessen oder indirekt ermittelt werden und mit dem Normvolumen oder davon abgeleiteten Parametern ein Korrekturfaktor mithilfe der Zentraleinheit bestimmt werden. Dieser Korrekturfaktor ist anschließend durch die Zentraleinheit auf ein Oszillationsprofil anwendbar, beispielsweise indem die Beträge der Amplituden in dem Oszillationsprofil mit dem Korrekturfaktor multipliziert werden und ein entsprechend korrigiertes Oszillationsprofil für eine Weiterverarbeitung oder eine Datenanzeige abgespeichert wird.

Neben einer Multiplikation der Amplituden des Oszillationsprofils mit einem Korrekturfaktor ist auch die Addition eines Korrekturwerts möglich, beispielsweise wenn die Amplituden nicht gestreckt oder gestaucht, sondern lediglich gleichmäßig verlängert oder verkürzt werden sollen.

Somit können mehrere Oszillationsprofile bzw. korrigierte Oszillationsprofile, die für unterschiedliche Probanden ermittelt wurden, miteinander verglichen werden und es ist sichergestellt, dass Abweichungen unter den Oszillationsprofilen sowie Auffälligkeiten innerhalb der einzelnen Oszillationsprofile zuverlässig analysiert werden können und eine absolute Aussagekraft bieten. Auch können somit mehrere Oszillationsprofile desselben Probanden mit einer hohen Zuverlässigkeit und Aussagekraft bereitgestellt und analysiert werden, ohne dass abweichende Aufpumpzustände der Manschetten etwa durch ein unregelmäßiges Aufpumpen oder wechselnde Ärzte eine Gegenüberstellung der Oszillationsprofile bedingt aussagekräftig machen können.

Diese und weitere Vorteile und Merkmale der Erfindung werden nachfolgend anhand von Beispielen unter Zuhilfenahme der begleitenden Figuren näher erläutert. Es zeigen:
**Figur 1** eine in skizzierter Form dargestellte Vorrichtung gemäß einer ersten Ausführungsform der Erfindung;
**Figur 2** ein in skizzierter Form dargestelltes, mit einer erfindungsgemäßen Vorrichtung erfasstes, Oszillationsprofil;
**Figur 3** ein weiteres in skizzierter Form dargestelltes, mit einer erfindungsgemäßen Vorrichtung erfasstes, Oszillationsprofil; und
**Figur 4** ein beispielhaftes Bildschirmfoto einer Anzeigeeinheit der Vorrichtung gemäß einer weiteren Ausführungsform der Erfindung.

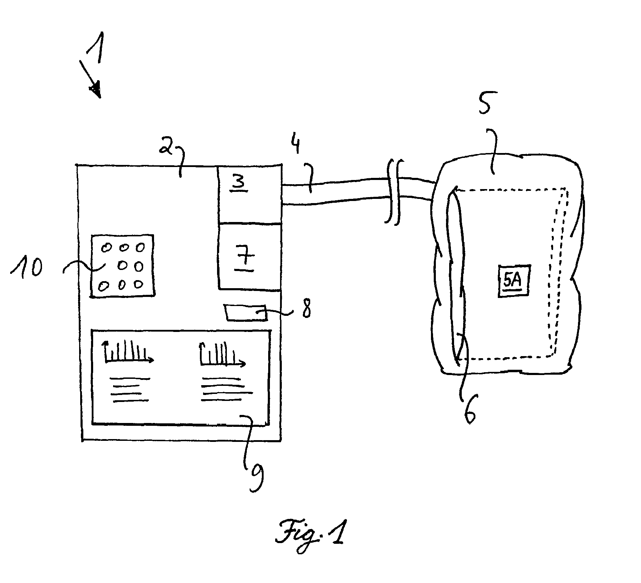

**Fig. 1** zeigt eine Skizze einer Vorrichtung 1 zum Erfassen von kardiologisch relevanten Parametern eines Probanden gemäß einer ersten Ausführungsform. Die Vorrichtung 1 weist eine Zentraleinheit 2 zum Messen von Blutdruckwerten einer Mehrzahl von aufeinander folgenden Pulsschlägen des Probanden auf. Dazu ist eine Pumpeinheit 3 der Zentraleinheit 2 über einen Luftschlauch 4 mit einer aufblasbaren bzw. aufpumpbaren Manschette 5 der Vorrichtung 1 verbunden. Die Manschette 5 weist einen Drucksensor 5A auf und hat eine Öffnung 6, durch die eine Extremität des Probanden hindurch gesteckt werden kann. Die Öffnung 6 ist mit gestrichelten Linien dargestellt. Die Pumpeinheit 3 kann ein Aufpumpen und ein Entleeren der Manschette 5 bewirken, da sie eine Luftpumpe und ein steuerbares Ventil aufweist.

Die Vorrichtung 1 weist ferner eine Anpasseinheit 7 auf, welche die Pumpeinheit 3 so ansteuern kann, dass die Manschette nach einem gewünschten Muster aufgepumpt oder abgepumpt bzw. entleert wird. Die Anpasseinheit ist bei dieser Ausführungsform ferner mit einem nicht abgebildeten Manschettendrucksensor in der Manschette 5 verbunden, um einen Manschettendruck bestimmen zu können. Ferner weist die Anpasseinheit 7 einen Zeitmesser auf, mithilfe dessen eine Zeitdauer eines Aufpump- oder Entleerungsvorgangs der Manschette 5 durch die Pumpeinheit 3 ermittelt werden kann. Daraus kann die Anpasseinheit ermitteln, welches Volumen an Luft in die Manschette eingegeben wurde bzw. zu einem bestimmten Zeitpunkt in der Manschette vorhanden ist.

Die Messdaten des Drucksensors 5A werden über eine in dem Luftschlauch 4 verlaufende elektrische Leitung an die Zentraleinheit 2 übermittelt und können in einer Speichereinheit 8 abgelegt werden. Die Zentraleinheit 2 ermittelt aus den Daten des Drucksensors 5A ein Oszillationsprofil, welches die Amplitudenwerte von Blutdruckwerten aufeinander folgender Pulsschläge enthält. Dabei wird ein oszillometrisches Messverfahren durchgeführt. Das Oszillationsprofil kann über eine Ausgabeeinheit 9 in Form eines Displays angezeigt werden. Außerdem ist eine Bedieneinheit 10 vorgesehen, mit deren Hilfe ein Benutzer der Vorrichtung 1 Eingaben oder Auswahlen vornehmen kann, um die Vorrichtung 1 zu steuern. Beispielsweise kann ein Benutzer die Anzeige von mehreren erfassten Oszillationsprofilen auswählen oder mehrere Oszillationsprofile auf der Anzeigeeinheit 9 gegenüberstellen.

Die Zentraleinheit 2 bzw. deren Anpasseinheit 7 kann anhand des ermittelten Volumens in der Manschette einen Korrekturfaktor bestimmen, der auf eine Profilhöhe, also eine durchschnittliche Amplitudenhöhe des Oszillationsprofils beispielsweise durch eine Multiplikationsoperation angewendet wird, um das Oszillationsprofil zu normieren und als ein korrigiertes Oszillationsprofil abzuspeichern.

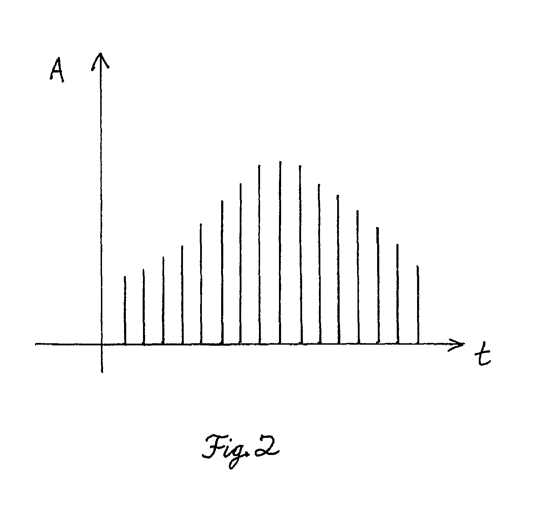

Fig. 2 zeigt ein in skizzierter Form dargestelltes Oszillationsprofil, das mit einer Vorrichtung gemäß Fig. 1 erfasst wurde. An der Ordinate sind Amplituden A von Blutdruckmesswerten aufeinander folgender Pulsschläge angetragen, während an der Abszisse deren Zeitverlauf t angetragen ist. Anfangs steigen die Amplitudenwerte an, erreichen etwa in der Mitte des Diagramms ihren Maximalwert und sinken anschließend wieder ab. Gut erkennbar ist in diesem Oszillationsprofil, dass die gemessenen Pulsschläge regelmäßig sind und keine Extrasystolen erfasst wurden. Außerdem sind keine Abweichungen in der Regelmäßigkeit des An- oder Abstiegs der Amplituden sichtbar, was auf ein Vorhofflimmern hindeuten könnte. Dieses Oszillationsprofil kann ausgegeben werden und insofern bei einer Diagnose des kardiologischen Zustands des entsprechenden Probanden Unterstützung leisten, als erkennbar ist, dass der Proband keine Pathologien bezüglich Extrasystolen oder ein Vorhofflimmern aufweist. Daneben können auf bekannte Weise die Pulsfrequenz, der systolische, der diastolische Blutdruck sowie der Mitteldruck aus dem Oszillationsprofil abgelesen werden.

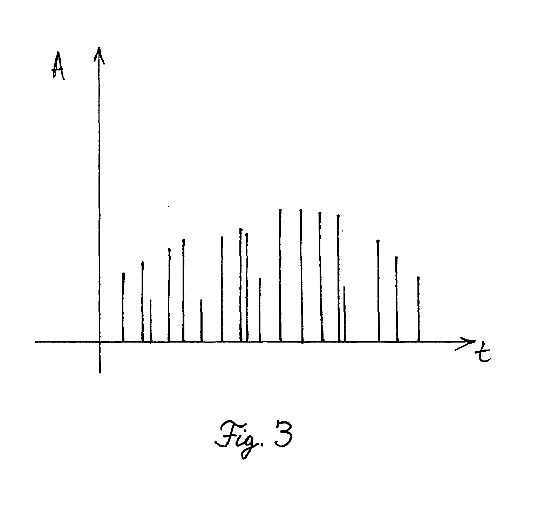

Fig. 3 zeigt ein weiteres, in skizzierter Form dargestelltes Oszillationsprofil, das mit einer Vorrichtung gemäß Fig. 1 erfasst wurde. Bei diesem Profil wird sofort deutlich, dass die Profilhöhe, also die gesamten Amplitudenwerte deutlich kleiner sind als bei dem Oszillationsprofil gemäß Fig. 2. Insbesondere weist das Diagramm Schwankungen im An- und Absteigen der Amplituden auf, was auf ein Vorhofflimmern des Herzens des Probanden hindeu ten kann. Ferner zeigt dieses Oszillationsprofil ungewöhnlich schnell aufeinander folgende Pulsschläge, sog. Extrasystolen, welche auf weitere Pathologien des Herzens oder des Gefäßsystems des Probanden hindeuten können.

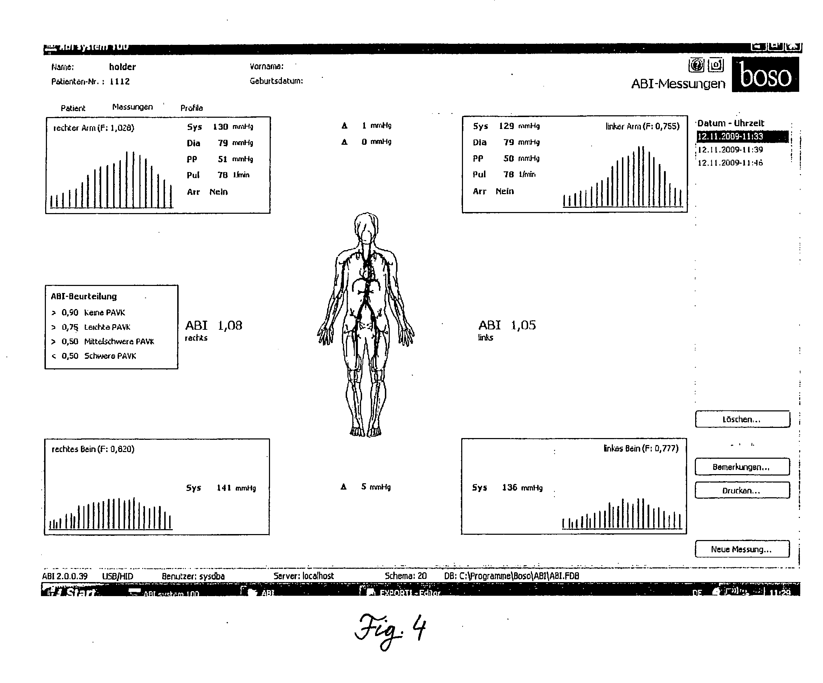

Fig. 4 zeigt ein Bildschirmfoto einer Anzeigeeinheit der Vorrichtung gemäß einer weiteren Ausführungsform der Erfindung, auf dem Messdaten angezeigt sind, die mithilfe der Vorrichtung erfasst wurden. An den Ecken der Figur sind vier Oszillationsprofile eines Probanden abgebildet, die an den beiden Armen bzw. an den beiden Beinen erfasst wurden. Beispielsweise zeigt das linke obere Diagramm das Oszillationsprofil für den rechten Arm, wie beispielsweise für die Fig. 2 oder 3 beschrieben. Daneben ist die Systole ,,Sys" mit 130 mmHg, die Diastole "Dia" mit 79 mmHg und der Pulsdruck "PP" mit 51 mmHg angegeben. Der Pulsdruck entspricht der Differenz aus Systole und Diastole und wurde durch die Zentraleinheit der Vorrichtung errechnet. Ferner ist der Puls ,,Pul" mit 78 1/min angegeben, was einer Herzfrequenz von 1,3 Hz entspricht. Mit ,,Arr Nein" wird angezeigt, dass während der Messung keine Unregelmäßigkeit der Pulsfrequenz von beispielsweise mehr als 25% vorlag. Wie oben beschrieben kann die Vorrichtung einen unregelmäßigen Pulsschlag erfassen und für eine Darstellung aufbereiten. In diesem Beispiel ist die Pulsfrequenz im Wesentlichen regelmäßig, so dass keine Unregelmäßigkeit auftritt.

Mit den Blutdruckdaten der vier Extremitäten wurden durch die Vorrichtung auch ein links- und ein rechtsseitiger ABI-Wert errechnet, also Knöchel-Arm-Index-Werte als Quotienten aus dem systolischen Wert einer Beinmessung geteilt durch den höheren systolischen Wert aus den beiden Oberarmmessungen. Hier werden die ABI-Werte mit 1,08 rechts und ABI 1,05 links angezeigt.

Ferner werden von der Vorrichtung errechnete Differenzwerte zwischen den Systolen der Arme mit einem Wert von 1 mmHg, zwischen den Diastolen der Arme mit einem Wert von 0 mmHg und zwischen den Systolen der Beine mit einem Wert von 5 mmHg angezeigt.

In der Mitte der Fig. 4 ist ein schematisches Blutgefäßsystem des Probanden dargestellt, in welchem kritische Werte mit bestimmten Farben veranschaulicht werden können. Die vorliegenden Messdaten lassen den Schluss zu, dass der Proband keine Pathologien oder sonstige kardiologische oder arterielle Auffälligkeiten aufweist. In diesem Fall kann das Blutsystem beispielsweise in blauer Farbe angezeigt werden. Werden beispielsweise Extrasystolen von der Vorrichtung erfasst, die sich dann auch in den Oszillationsprofilen widerspiegeln, kann etwa das Herz in dem schematischen Blutsystem in roter Farbe angezeigt werden. Wenn dagegen lediglich an einer Extremität eine Unregelmäßigkeit im entsprechenden Oszillationsprofil aufweist, kann das Gefäßsystem der entsprechenden Extremität in roter Farbe dargestellt werden, während das restliche System blau erscheint.

Somit können die durch die Vorrichtung gemessenen und errechneten kardiologischen Parameter auf eine sehr anschauliche Weise auf der Anzeigeeinheit angezeigt werden und einem Benutzer oder dem Probanden eine wertvolle Unterstützung für eine Diagnose bzw. einen medizinischen Befund liefern.

## Patentansprüche

1. Vorrichtung zum Erfassen von kardiologisch relevanten Parametern eines Probanden, mit einer an einer Extremität des Probanden anlegbaren Manschette und einem Drucksensor, **gekennzeichnet durch**
eine mit dem Drucksensor verbundene Zentraleinheit zum Messen von Blutdruckwerten einer Mehrzahl von aufeinander folgenden Pulsschlägen des Probanden mittels eines oszillometrischen Messprinzips und zum Erstellen eines Oszillationsprofils mit einer Serie der entsprechenden Blutdruckwerte.

2. Vorrichtung nach Anspruch 1, **gekennzeichnet durch** eine Speichereinheit zum Speichern des Oszillationsprofils mit den gemessenen Blutdruckwerten.

3. Vorrichtung nach einem der Ansprüche 1 oder 2, **gekennzeichnet durch** eine Anzeigeeinheit zum grafischen Anzeigen des Oszillationsprofils an einen Benutzer.

4. Vorrichtung nach einem der Ansprüche 1 oder 2, **gekennzeichnet durch** eine Ausgabeeinheit zum Ausgeben des Oszillationsprofils an eine externe Einheit.

5. Vorrichtung nach einem der vorangehenden Ansprüche, **gekennzeichnet durch**
eine Anpasseinheit zum Bestimmen eines Volumens in der Manschette und zum Normieren einer Amplitudenhöhe des Oszillationsprofils entsprechend des Volumens.

6. Vorrichtung nach Anspruch 5, **dadurch gekennzeichnet, dass** die Anpasseinheit dazu geeignet ist, einen von dem Volumen abhängigen Korrekturfaktor zu errechnen und die Amplitudenhöhe des Oszillationsprofils damit zu multiplizieren, um ein korrigiertes Oszillationsprofil zu erstellen.

7. Vorrichtung nach einem der Ansprüche 5 oder 6, **dadurch gekennzeichnet, dass** die Zentraleinheit die Anpasseinheit aufweist.

8. Vorrichtung nach einem der Ansprüche 5 bis 7, **gekennzeichnet durch** einen Volumensensor zum Messen des Volumens in der Manschette.
